(19) Europäisches Patentamt
European Patent Office
Office européen des brevets

(11) Publication number: **0 092 487**
**B1**

(12) **EUROPEAN PATENT SPECIFICATION**

(45) Date of publication of patent specification: **07.10.87**

(51) Int. Cl.$^4$: **C 07 D 207/34**

(21) Application number: **83400773.4**

(22) Date of filing: **19.04.83**

(54) **Process for the preparation of 3-carboxy-1,4-dimethylpyrrol-2-acetic acid.**

(30) Priority: **21.04.82 IT 2084682**

(43) Date of publication of application:
**26.10.83 Bulletin 83/43**

(45) Publication of the grant of the patent:
**07.10.87 Bulletin 87/41**

(84) Designated Contracting States:
**BE DE FR GB NL**

(56) References cited:
**GB-A-2 034 304**
**US-A-3 865 840**
**US-A-4 363 918**

(73) Proprietor: **Montedison S.p.A.**
**31, Foro Buonaparte**
**I-20121 Milan (IT)**

(72) Inventor: **Bottaccio, Giorgio**
**16 Via Manin**
**Novara (IT)**
Inventor: **Campolmi, Stefano**
**27 Via Andrea Costa**
**Novara (IT)**
Inventor: **Carletti, Vittorio**
**7 Largo Europa-Meda**
**Milan (IT)**
Inventor: **Marchi, Marcello**
**11 Viale Allegra**
**Novara (IT)**

(74) Representative: **Hirsch, Marc-Roger**
**Cabinet Hirsch 34 rue de Bassano**
**F-75008 Paris (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention concerns a process for the preparation of 3-carboxy-1,4-dimethylpyrrol-2-acetic acid having the formula (I):

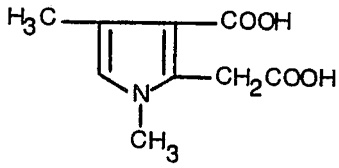

(I)

In particular the present invention concerns a process for the preparation of 3-carboxy-1,4-dimethylpyrrol-2-acetic acid by condensation of a halo-acetone and of methylamine with acetone-bicarboxylic acid or its alkaline salts.

According to an alternate aspect of this invention, it relates to a process for the preparation of 3-carboxy-1,4-dimethylpyrrol-2-acetic acid through the above mentioned condensation using the alkaline salts of acetone-bicarboxylic acid, obtained in their turn by reaction of $CO_2$ and alkaline phenates with acetone.

The 3-carboxy-1,4-dimethylpyrrol-2-acetic acid thus obtained represents an important intermediate for the synthesis of organic compounds in general, more particularly it finds a useful application in the preparation of the compounds belonging to the class of alkanoic 5-aryloyl pyrrol acids, known in the pharmaceutical industry as anti-inflammatory agents, and more particularly in the preparation of 5-(p-chloro-benzoyl)-1,4-dimethylpyrrol-2-acetic acid having the formula (II):

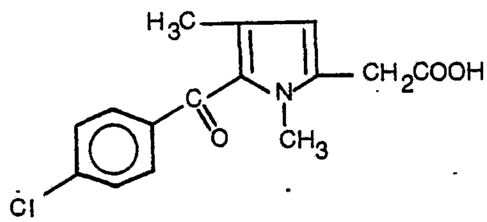

(II)

also known as "ZOMEPIRAC", acting effectively as anti-inflammatory agent.

It is known from USP 3865840 to prepare compounds of the class of derivatives of 3-carboxy-1,4-dialkylpyrrol-2-alkanoic acids, in general in the form of esters.

Such a process consists in reacting an alkylamine with chloro-acetone and with an alkyl ester of acetone-bicarboxylic acid in an aqueous or aqueous-organic reaction medium (for instance dichloroethane and $H_2O$) at substantially room temperature.

However, the above said process, with regard to the preparation of the 5-aryloyl-pyrrol-alkanoic derivatives of main interest, has the drawback that it prepares said derivatives in the form of alkylic esters, which, although, on the one hand, possess a greater stability in comparison with the salts or acids and, thus, are easier to preserve and transport etc., on the other hand, make necessary the saponification of said derivatives, both with regard to the products of the synthesis in general as well as, in particular, in the case of their further utilization as pharmaceutical products in acid form.

Furthermore, the alkyl esters of acetone-dicarboxylic acid, used according to the technique known per se, are relatively expensive while, on the other hand, they require, as indicated herein-above, to be saponified in the end product.

This final operation will thus be expensive and time-consuming with respect to the essence of the preparatory process of the 5-aryloyl-pyrrolalkanoic acid derivatives of main interest.

Thus, the present invention relates to providing a process for the preparation of 3-carboxy-1,4-dimethylpyrrol-2-acetic acid of formula (I) as herein-above defined, a simple and inexpensive process that overcomes the drawback to be found in the methods of the previously considered prior art.

Furthermore, the present invention, concerns providing a process for the preparation of 3-carboxy-1,4-dimethylpyrrol-2-acetic acid (I) as herein-above defined, according to a method that can be advantageously combined with an already available technology which allows the preparation of acid (I) starting from acetone, $CO_2$ and alkaline phenates, particularly inexpensive raw materials.

These and further objects, that will appear evident to the man skilled in the art from the following description, will be attained according to this invention by a process for the preparation of 3-carboxy-1,4-diméthylpyrrol-2-acetic acid of formula (I), as previously herein-above defined, characterized in that a carboxylated compound chosen from between acetone-bicarboxylic acid and its alkaline salts, is made to react in an aqueous medium with methylamine and a halo-acetone, at substantially room temperature.

Described in further detail, the process object of the present invention consists in adding to the acetone-bicarboxylic acid or to one of its alkaline salts, the methylamine in an aqueous medium, at a temperature preferably comprised between about 5°C and 10°C, and by then adding gradually to the same reaction medium the halo-acetone at a temperature lower than about 40°C, and by subsequently carrying on the reaction without the need of any thermoregulation.

The process may be schematically represented by the following equation:

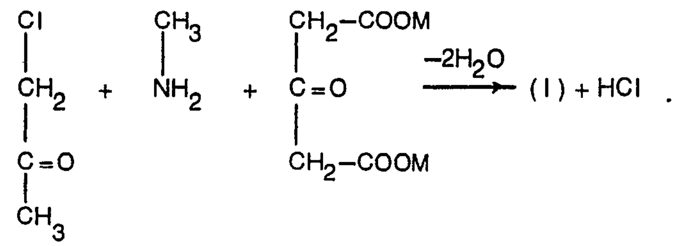


In this equation chloro-acetone has been introduced as a halo-acetone; similar results can be obtained with the use of bromo-acetone. M represents a hydrogen atom or an atom of an alkaline metal, preferably chosen between Na and K; in this latter case, acide (I) is obtained in the form of its corresponding alkaline salt.

The reactants are introduced according to substantially equimolar ratios corresponding to the stoichimetric reaction.

In the case of M=H, i.e., in the case of the use of acetone-bicarboxylic acid, an excess of methylamine is required in order to salify the free acid functions.

The temperature during the reaction is maintained at substantially room temperatures (below about 40°C).

Better results are obtained, as previously indicated, when the temperature is gradually brought from about initially 5°—10°C, at the moment of the addition of the methylamine to the aqueous acetone-bicarboxylic acid or its aqueous alkaline salt, up to room temperature (but still below about 40°C) during the addition of the halo-acetone.

Since the reaction is exothermic, the control of temperature of the reaction can be facilitated when the addition of halo-acetone occurs in a gradual way, under external cooling of the reactor.

The reaction times depend on the parametric conditions adopted in the operation: at any event, they are comprised within a time interval of between about 2 and 3 hours, sufficient to complete the entire reaction.

At the end of the reaction the resulting product is separated and purified according to substantially conventional techniques.

For instance, at the end of the reaction, the reactive mixture is poured in the cold state into an aqueous solution of hydrochloric acid until a pH value comprised between 5 aand 6 is reached and subsequently the aqueous phase is extracted by using ethyl ether, then further acidified, etc.

According to a particularly effective and economic embodiment, the process object of this invention, may be applied directly to the alkaline salts of acetone-carboxylic acid such as obtained according to the known process for their preparation initially using acetone, $CO_2$ and alkaline phenates.

Said process is briefly summarized in herein-under.

To a solution of alkaline phenate in the chosen aprotic solvent and preventively saturated with carbon dioxide, is added under vigorous stirring, at a pre-fixed temperature, the acetone in determined proportions. The reaction mixture is then subjected to stirring under a carbon dioxide atmosphere until completion of the reaction. At this point the determined quantity of water is added.

The alkaline salts of the produced acetoacetic and 3-ketoglutaric acids, together with the bicarbonate derived from the phenate in excess, precipitate in a crystalline form and are then filtered and washed with a solvent (benzene, petrolum ether, chloroform).

The acid may be freed of the salt by means of techniques known per se, by acidification with diluted sulphuric acid followed by extraction with ethyl ether, etc.

According to a typical embodiment, the process object of this invention is achieved in the following way.

Into a reactor fitted with temperature regulating and reactant-dosing systems, is introduced an aqueous methylamine solution. Thereafter is added acetone-bicarboxylic acid at a temperature comprised between about 5° and 10°C; or alternatively, is added the raw mixture of the alkaline salt of acetone-carboxylic acid, obtained according to the process of carboxylation of the acetone with phenates, as described herein-above.

To the mixture obtained according to any one of the above said alternatives, halo-acetone (chloro-acetone) is gradually added while maintaining the temperature below 40°C, and thereafter the reaction is carried out at substantially room temperature for about 2 hours. At the end of the reaction 3-carboxy-1,4-dimethylpyrrol-2-acetic acid produced is separated and its purification carried out as indicated above, etc.

This process, due to its simple and mild operational conditions, has proved to be particularly convenient.

One particular advantage is the possibility of directly using as a source of alkaline salts of acetone-bicarboxylic acid the raw mixture that contains them as produced in the carboxylation process of the acetone with $CO_2$ and alkaline phenates. Although containing said raw mixture, other organic alkaline salts (aceto-acetates) and inorganic alkaline salts (carbonates and bicarbonates), the results obtained remain pratically uninfluenced by these impurities.

In this way it is made possible, by integrating them into the present invention, the best exploitation of an already available technology that supplies in a directly and economically usable form, the alkaline salts of acetone-bicarboxylic acid necessary for the process object of the present invention.

The invention will now be further described in the following examples, given for non-limitative illustration.

Example 1

Into a 500 cc flask fitted with a stirrer, a thermometer, a dripping funnel and a loading system for the reactants, were introduced: 35 cc of water and 90 cc of an aqueous 35% by weight solution of methylamine. To this solution were then added 16 grams of acetone-bicarboxylic acid at a temperature of between 5° and 10°C. Thereafter, over a period of 10 minutes, 15.5 grams of chloro-acetone, were dripped into the reaction mixture, keeping the temperature below about 40°C. The mixture was then kept under stirring for 2 hours at room temperature.

The reaction mixture was poured into a cold aqueous solution of an aqueous hydrochloric acid diluted (to about 10%) until it attained a pH value of between 5 and 6. The aqueous phase was then extracted with two portions of 100 cc each of ethyl ester, and it was further acidified with hydrochloric acid diluted (about 10%) to a pH = 1. A solid precipitated which was filtered, washed with water and then dried. 8 g of a white product (melting point = 208—211°C) were obtained which was identified as 3-carboxy-1,4-dimethylpyrrol-2-acetic acid.

Example 2

65 grams of the filtration cake, containing about 18 g of acetone-bicarboxylic acid in the form of bisodium salt, obtained by the carboxylation reaction of acetone with $CO_2$ and sodium 2-terbutyl-p-cresolate in tetrahydrofurane, according to the operational procedures described in Italain Patent n° 1,034,702 (example 18), were introduced suspended in 105 cc of water, into a 500 cc reactor fitted with a stirrer, a thermometer, a dripping funnel and a reactant-loading system. 90 cc of an aqueous 35% by weight solution of methylamine, at a temperature of between 5°C and 10°C, were dripped in.

Over a period of 10 minutes, 15.5 grams of chloro-acetone were added, maintaining the temperature below 40°C. This mixture was then kept under stirring for 2 hours at room temperature.

At the end of this period, the reaction mixture was treated as in Example 1, about 10 grams of a white product is obtained (melting point comprised between 210° and 213°C), identified as 3-carboxy-1,4-dimethylpyrrol-2-acetic acid.

**Claims**

1. Process for the preparation of 3-carboxy-1,4-dimethylpyrrol-2-acetic acid having the formula (I):

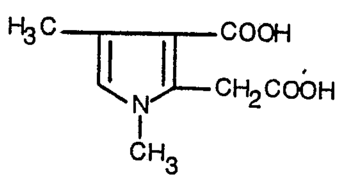

(I)

characterized in that a carboxylic compound chosen from among acetone-bicarboxylic acid and its alkaline salts, is made to react in an aqueous medium with methylamine and a halo-acetone at substantially room temperature.

2. Process according to claim 1, characeterized in that the haloacetone is chosen from between chloro-acetone and bromo-acetone.

3. Process according to claim 1, characeterized in that the alkaline salts of acetone-bicarboxylic acid are chosen from among the Na and K salts.

4. Process according to claim 1, characeterized in that the reactants are introduced according to substantially stoichiometric ratios.

5. Process according to claim 1, characeterized in that the reaction is preferably conducted by adding to the acetone-bicarboxylic acid or to its alkaline salts, the methylamine at a temperature comprised between about 5° and 10°C, and then by proceeding with the addition of the haloacetone at room temperature, and in any event below about 40°C.

6. Process according to claim 1, characeterized in that said process is carried out over a period of between 2 and 3 hours.

7. Process according to claim 1, characterized in that to operate said process is used an alkaline salt chosen from among the Na and K salts of the raw acetone-bicarboxylic acid as obtained by the reaction of acetone with $CO_2$ and alkaline phenates in an organic medium.

**Patentansprüche**

1. Verfahren zur Herstellung von 3-Carboxyl-1,4-dimethylpyrrol-2-essigsäure mit der Formel (I):

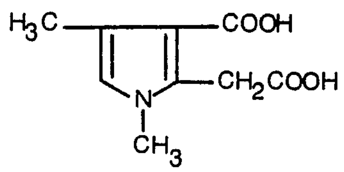

(I)

dadurch gekennzeichnet, dass man eine Carboxylverbindung der Acetoncarboxylsäure oder ihrer Alkalisalze in einem wässerigen Medium mit Methylamin und einem Halogenaceton bei einer wesentlich der Zimmertemperatur entsprechenden Temperatur reagieren lässt.

2. Verfahren nach Anspruch 1, dadurch gekennzeichnet, dass das Halogenaceton Chloraceton oder Bromaceton ist.

3. Verfahren nach Anspruch 1, dadurch gekennzeichnet, dass die Alkalisalze der Acetonbicarboxylsäure Natrium- oder Kaliumsalze sind.

4. Verfahren nach Anspruch 1, dadurch gekennzeichnet, dass die Reagenzien in wesentlich stoechiometrischen Proportionen eingesetzt werden.

5. Verfahren nach Anspruch 1, dadurch gekennzeichnet, dass die Reaktion vorzugsweise durch Hinzufügung des Methylamins zur Acetonbicarboxylsäure oder deren Alkalisalzen bei einer Temperatur von etwa 5° bis 10°C durchgeführt wird, während das Halogenaceton soddan bei Zimmertemperatur, und jedenfalls bei einer Temperatur von weniger als etwa 40°C, zugegeben wird.

6. Verfahren nach Anspruch 1, dadurch gekennzeichnet, dass es während einer Dauer von etwa 2 bis 3 Stunden durchgeführt wird.

7. Verfahren nach Anspruch 1, dadurch gekennzeichnet, dass zwecks Durchführung desselben ein Natrium- oder Kaliumsalz der rohen Acetonbicarboxylsäure gewählt wird, welche durch die Reaktion von Aceton mit $CO_2$ und alkalischen Phenaten in einem organischen Medium erzeugt wird.

**Revendications**

1. Procédé de préparation de l'acide 3-carboxyl-1,4-diméthylpyrrol-2-acétique de formule (I):

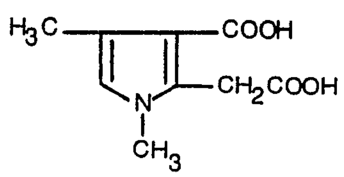

(I)

caractérisé en ce qu'on fait réagir un composé carboxyle du groupe constitué par l'acide acétone-bicarboxylique et ses sels alcalins, dans un milieu aqueux avec la méthylamine et une halo-acétone à une température correspondant sensiblement à la température ambiante.

2. Procédé selon la revendication 1, caractérisé en ce que l'acétone halogéné est choisi parmi les membres du groupe constitué par la chloro-acétone et la bromo-acétone.

3. Procédé selon la revendication 1, caractérisé en ce que les sels alcalins de l'acide acétone-bicarboxylique sont choisis parmi les membres du groupe constitué par les sels de sodium et les sels de potassium.

4. Procédé selon la revendication 1, caractérisé en ce qu'on introduit les réactifs dans des proportions sensiblement stoechiométriques.

5. Procédé selon la revendication 1, caractérisé en ce que la réaction est mise en oeuvre, de préférence, par addition à l'acide acétone-bicarboxylique ou à ses sels alcalins, de la méthylamine, à une température comprise entre environ 5° et 10°C, et par addition consecutive de 1 halo-acétone, à température ambiante, et dans tous les cas inférieure à environ 40°C.

6. Procédé selon la revendication 1, caractérisé en ce qu'il est mis en oeuvre pendant une période comprise entre environ 2 et 3 heures.

7. Procédé selon la revendication 1, caractérisé en ce qu'afin de le mettre en oeuvre, on utilise un sel alcalin choisi parmi les sels de sodium et les sels de potassium de l'acide acétone-carboxylique brut tel qu'obtenu par la réaction de l'acétone avec le $CO_2$ et des phénates alcalins, dans un milieu organique.